# EUROPÄISCHE PATENTSCHRIFT

(11) **EP 0 930 367 B1**
(45) Veröffentlichungstag und Bekanntmachung des Hinweises auf die Patenterteilung: **15.12.2004**
(21) Anmeldenummer: 98124402.3
(22) Anmeldetag: 21.12.1998
(51) Int. Cl.: C12N 15/80, C12N 15/60, C12N 15/54, C12P 25/00, C12N 1/15

(54) **Pilz der Gattung Ashbya zur Herstellung von Riboflavin**
Fungi of the genus Ashbya for the production of riboflavin
Champignons du genre Ashbya pour la préparation de riboflavine

(30) Priorität: 22.12.1997 DE 19757180; 07.09.1998 DE 19840709
(43) Veröffentlichungstag der Anmeldung: 21.07.1999
(73) Patentinhaber: BASF AKTIENGESELLSCHAFT, 67056 Ludwigshafen (DE); Forschungszentrum Jülich GmbH, 52425 Jülich (DE)
(72) Erfinder: Monschau, Nicole, 41199 Mönchengladbach (DE); Stahmann, Klaus-Pter, 52428 Jülich (DE); Sahm, Hermann, 52428 Jülich (DE); Zelder, Oskar, 67346 Speyer (DE)
(74) Vertreter: Fitzner, Uwe, Dr.

(56) Entgegenhaltungen:
- EP-A- 0 337 502
- US-A- 2 876 169
- MONSCHAU,N. ET AL.: "Identification of Saccharomyces cerevisiae GLY1 gene as a threonine aldolase: a key enzyme in glycine biosynthesis" FEMS MICROBIOL. LETT., Bd. 150, Nr. 1, 1. Mai 1997 (1997-05-01), Seiten 55-60, XP002118341
- MCNEIL,J.B. ET AL.: "Cloning and Molecular Characterization of three genes, including two genes encoding serine hydroxymethyltransferase, whose inactivation is required to render yeast auxotrophic for glycine" J.BIOL.CHEM., Bd. 269, Nr. 12, 25. März 1994 (1994-03-25), Seiten 9155-9165, XP002118342
- MONSCHAU,N. ET AL.: "Threonine aldolase overexpression plus threonine supplementation enhanced riboflavin production in Ashbya gossypii" APPL. ENVIRON. MICROBIOL. , Bd. 64, Nr. 11, 1. November 1998 (1998-11-01), Seiten 4283-4290, XP002118343 -& DATABASE GENEMBL [Online] Database accession no. AJ005442 XP002118559

## Beschreibung

Die vorliegende Erfindung betrifft einen genetisch veränderten Pilz der Gattung Ashbya zur Herstellung von Riboflavin.

Das Vitamin B₂, auch Riboflavin genannt, ist für Mensch und Tier essentiell. Bei Vitamin-B₂-Mangel treten Entzündungen der Mund- und Rachenschleimhäute, Risse in den Mundwinkeln, Juckreiz und Entzündungen in den Hautfalten u.a. Hautschäden, Bindehautentzündungen, verminderte Sehschärfe und Trübung der Hornhaut auf. Bei Säuglingen und Kindern können Wachstumsstillstand und Gewichtsabnahme eintreten. Das Vitamin B₂ hat daher wirtschaftliche Bedeutung insbesondere als Vitaininpräparat bei Vitaminmangel sowie als Futtermittelzusatz. Daneben wird es auch als Lebensmittelfarbstoff, beispielsweise in Mayonnaise, Eiscreme, Pudding etc., eingesetzt.

Die Herstellung von Riboflavin erfolgt entweder chemisch oder mikrobiell. Bei den chemischen Herstellungsverfahren wird das Riboflavin in der Regel in mehrstufigen Prozessen als reines Endprodukt gewonnen, wobei allerdings auch relativ kostspielige Ausgangsprodukte - wie beispielsweise D-Ribose - eingesetzt werden müssen. Daher kommt die chemische Synthese des Riboflavins nur für solche Anwendungszwecke in Betracht, für die reines Riboflavin notwendig ist, wie z.B. in der Humanmedizin.

Eine Alternative zur chemischen Herstellung des Riboflavins bietet die Herstellung dieses Stoffes durch Mikroorganismen. Die mikrobielle Herstellung des Riboflavins eignet sich insbesondere für solche Fälle, in denen eine hohe Reinheit dieser Substanz nicht erforderlich ist. Dies ist beispielsweise dann der Fall, wenn das Riboflavin als Zusatz zu Futtermittelprodukten eingesetzt werden soll. In solchen Fällen hat die mikrobielle Herstellung des Riboflavins den Vorteil, daß diese Substanz in einem einstufigen Prozeß gewinnbar ist. Auch können als Ausgangsprodukte für die mikrobielle Synthese nachwachsende Rohstoffe, wie beispielsweise pflanzliche Öle, eingesetzt werden.

Die Herstellung von Riboflavin durch Fermentation von Pilzen wie Ashbya gossypii oder Eremothecium ashbyi ist bekannt (The Merck Index, Windholz et al., eds. Merck & Co., Seite 1183, 1983, A. Bacher, F. Lingens, Augen. Chem. 1969, S. 393 und US Patentschrift US 2 876 169); aber auch Hefen, wie z.B. Candida oder Saccharomyces, und Bakterien wie Clostridium, sind zur Riboflavinproduktion geeignet).

Zudem sind Verfahren mit der Hefe Candida famata beispielsweise in der US 05231007 beschrieben.

Riboflavin-überproduzierende Bakterienstämme sind beispielsweise in der EP 405370, GB 1434299, DE 3420310 und EP0821063 beschrieben, wobei die Stämme durch Transformation der Riboflavin-Biosynthese-Gene aus Bacillus subtilis erhalten wurden. Diese Prokaryonten-Gene waren aber für ein rekombinantes Riboflavin-Herstellungsverfahren mit Eukaryonten wie Saccharomyces cerevisiae oder Ashbya gossypii ungeeignet. Daher wurden gemäß der WO 93/03183 für die Riboflavin-Biosynthese spezifische Gene aus einem Eukaryonten, nämlich aus Saccharomyces cerevisiae, isoliert, um damit ein rekombinantes Herstellungsverfahren für Riboflavin in einem eukaryontischen Produktionsorganismus bereitzustellen. Derartige rekombinante Herstellungsverfahren haben für die Riboflavin-Produktion jedoch dann keinen oder nur begrenzten Erfolg, wenn die Bereitstellung von Substrat für die an der Riboflavin-Biosynthese spezifisch beteiligten Enzyme unzureichend ist.

1967 fand Hanson (Hanson AM, 1967, in Microbial Technology, Peppler, HJ, pp.222-250 New York), daß der Zusatz der Aminosäure Glycin die Riboflavin-Bildung von Ashbya gossypii steigert. Ein derartiges Verfahren ist jedoch nachteilig, weil Glycin ein sehr teurer Rohstoff ist. Aus diesem Grunde war man bestrebt, durch Herstellung von Mutanten die Riboflavin-Produktion zu optimieren.

Aus der deutschen Patentschrift 19525281 ist ein Verfahren zur Herstellung von Riboflavin bekannt bei dem Mikroorganismen kultiviert werden, die resistent gegenüber auf Isocitrat Lyase hemmend wirkenden Substanzen sind.

Aus der deutschen Offenlegungsschrift 19545468.5-41 ist ein weiters Verfahren zur mikrobiellen Herstellung von Riboflavin bekannt, bei dem die Isocitratlyase-Aktivität oder die Isocitratlyase-Genexpression eines Riboflavin produzierenden Mikroorganismus erhöht ist. Aber auch im Vergleich zu diesen Verfahren besteht noch ein Bedarf, zu einer weiteren Optimierung der Riboflavin-Herstellung.

Aufgabe der vorliegenden Erfindung ist es demgemäß, einen ein- oder mehrzelligen Organismus, vorzugsweise einen Pilz, für die biotechnische Herstellung von Riboflavin zur Verfügung zu stellen, der eine weitere Optimierung der Riboflavin-Bildung ermöglicht. Insbesondere sollte ein Organismus zur Verfügung gestellt werden, der unter Einsparung von Rohstoffen für die Herstellung von Riboflavin geeignet ist und damit eine Produktion ermöglicht, die gegenüber dem bisherigen Stand der Technik wirtschaftlicher ist. Vor allem soll der Organismus eine im Vergleich zu den bisherigen Organismen erhöhte Riboflavin-Bildung ohne Zusatz von Glycin erlauben.

Diese Aufgabe wird durch einen genetisch veränderten ein- oder mehrzelligen Pilz der Gattung Ashbya gelöst, der durch eine erhöhte Synthese der Threonin-Aldolase, kodiert durch eine Nukleotidsequenz gemäß SEQ ID No. 1 oder deren Allelvariationen eine gegenüber seiner endogenen Threonin-Aldolase-Aktivität erhöhte katalytische Aktivität diese Enzyms aufweist.

Gegenstand der Erfindung ist ein genetisch veränderter Pilz der zuvor genannten Art, der sich dadurch auszeichnet, dass seine Riboflavinsyntheseleistung ohne externe Zufuhr von Glycin mindestens gleich derjenigen eines Wildtyps der Species Ashbya ATCC 10895 ist, der unter Standardbedingungen unter Zugabe von 6 g/l externen Glycin gezüchtet wird.

Züchtung unter Standardbedingungen bedeutet die Kultivierung in 500 ml Schüttelkolben mit zwei Schikanen bei 120 RpM und 30 ° C. Als Medium werden pro Kolben 50 ml einer Lösung von 10g/l Hefeextrakt entweder mit 10 g/l Glucose oder mit 10g/l Sojaöl eingesetzt. Die Beimpfung erfolgt mit 1 % einer 16 h Kultur unter gleichen Bedingungen.
Das Ziel dieser angestrebten Veränderung des intrazellulären Glycinstoffwechsels kann mit den bekannten Methoden der Stammverbesserung von Pilzen erreicht werden. D.h. im einfachsten Falle lassen sich entsprechende Stämme nach der in der Mikrobiologie üblichen Selektion mittels Screening herstellen. Ebenso ist die Mutation mit anschließender Selektion einsetzbar. Die Mutation kann hierbei sowohl mittels chemischer als auch mittels physikalischer Mutagenese ausgeführt werden. Eine weitere Methode ist die Selektion und Mutation mit anschließender Rekombination. Schließlich lassen sich die erfindungsgemäßen Pilze mittels Genmanipulation herstellen

In der vorliegenden Erfindung gehört der genetisch veränderte ein- oder mehrzellige Pilz zur Gattung Ashbya. Ein bevorzugter genetisch veränderter ein- oder mehrzelliger Pilz ist Ashbya gossypii.

Erfindungsgemäß wird der genetisch veränderte Pilz derart verändert, daß er intrazellulär Glycin in einer Menge erzeugt, die größer als sein Bedarf für die Aufrechterhaltung seines Metabolismus ist. Diese Erhöhung der intrazellulären Glycinerzeugung läßt sich erfindungsgemäß vorzugsweise dadurch erreichen, daß ein genetisch veränderter Pilz hergestellt wird, bei dem die Enzymaktivität der Threonin-Aldolase erhöht ist. Eine erhöhte Enzymaktivität der Threonin-Aldolase Kann durch Erhöhung der Enzymsynthese, beispielsweise durch Genamplifikation oder durch Ausschaltung von Faktoren, die die Enzym-Biosynthese reprimieren, hervorgerufen werden.

Die Threonin-Aldolase-Genexpression kann durch Einbau von Threonin-Aldolase-Genkopien und/oder durch Verstärkung regulatorischer Faktoren, die die Threonin-Aldolase-Genexpression positiv beeinflussen, erreicht werden. So kann eine Verstärkung regulatorischer Elemente vorzugsweise auf Transcriptionsebene erfolgen, indem insbesondere die Transcriptionssignale erhöht werden. Daneben ist aber auch eine Verstärkung der Translation möglich, indem beispielsweise die Stabilität der m-RNA verbessert wird.

Zur Erhöhung der Genkopienzahl kann beispielsweise das Threonin-Aldolase-Gen in ein Genkonstrukt bzw. in einen Vektor eingebaut werden, der vorzugsweise dem Threonin-Aldolase-Gen zugeordnete regulatorische Gensequenzen enthält, insbesondere solche, die die Genexpression verstärken. Anschließend wird ein Riboflavin-produzierender Mikroorganismus, mit dem das Threonin-Aldolase-Gen enthaltenden Genkonstrukt transformiert. Erfindungsgemäß kann die Überexpression der Threonin-Aldolase auch durch Austausch des Promotors erzielt werden. Hierbei ist es möglich, die höhere enzymatische Aktivität alternativ durch Einbau von Genkopien oder durch Austausch des Promotors zu erzielen. Gleichermaßen ist es jedoch auch möglich, durch gleichzeitigen Austausch des Promotors und Einbau von Genkopien die gewünschte Änderung der Enzymaktivität zu erzielen.

Da bei einem derart genetisch veränderten Pilz das Threonin limitierend wirkt, ist bei Einsatz der erfindungsgemäßen Zelle die Zufütterung von Threonin erforderlich. Die bessere Aufnahme und nahezu quantitative Umsetzung des Threonins zu Glycin führen zu einer überraschend hohen Steigerung der Riboflavin-Bildung, wie sie bisher durch Zufütterung von Glycin nicht erreichbar war. Alternativ kann die endogene Threoninbildung des genetisch veränderten Pilzes z.B. durch Ausschaltung der Feedback-Resistenz der Aspartatkinase erhöht werden.

Das Threonin-Aldolase-Gen wird vorzugsweise aus Mikroorganismen, der Spezies Ashbya gossypii.

Für die Isolierung des Gens kommen aber auch alle weiteren Organismen, deren Zellen die Sequenz zur Bildung der Threonin-Aldolase enthalten, also auch pflanzliche und tierische Zellen, in Betracht. Die Isolierung des Gens kann durch homologe oder heterologe Komplementation einer im Threonin-Aldolase-Gen defekten Mutante oder auch durch heterologes Probing oder PCR mit heterologen Primern erfolgen. Zur Subklonierung kann das Insert des komplementierenden Plasmids anschließend durch geeignete Schritte mit Restriktionsenzymen in der Größe minimiert werden. Nach Sequenzierung und Identifizierung des putativen Gens erfolgt eine paßgenaue Subklonierung durch Fusions-PCR. Plasmide, die die so erhaltenen Fragmente als Insert tragen, werden in die Threonin-Aldolase-Gen-Defekte Mutante eingebracht, die auf Funktionalität des Threonin-Aldolase-Gens getestet wird. Funktionelle Konstrukte werden schließlich zur Transformation eines Riboflavin-Produzenten eingesetzt.

Nach Isolierung und Sequenzierung sind die Threonin-Adolase-Gene mit Nukleotidsequenzen erhältlich, die für die angegebene Aminosäure-Sequenz. Gegenstand der vorliegenden Erfindung ist eine Threonin-Aldolase mit einer für die in SEQ ID No. 2 angegebenen Aminosäuresequenz. Ferner ist eine Nukleotid gemäß SEQ ID No. 1 kodierend für eine Threonin-Aldolase umfasst. Die erfindungsgemäße Nukleotidsequenz weist dabei eine Kodierregion für ein Threonin-Aldolase-Gen von Nukleotid 1 bis 1149 gemäß SEQ ID No. 1 auf.

Dem Threonin-Aldolase-Gen ist insbesondere ein Promotor der Nukleotidsequenz von Nukleotid -1231 bis -1 gem. der SEQ ID No. 1 vorgeschaltet. So kann beispielsweise dem Gen ein Promotor vorgeschaltet sein, der sich von dem Promotor mit der angegebenen Nukleotidsequenz durch ein oder mehrere Nukleotidaustausche, durch Insertion und/oder Deletion unterscheidet, ohne daß aber die Funktionalität bzw. die Wirksamkeit des Promotors beeinträchtigt wird. Des weiteren kann der Promotor durch Veränderung seiner Sequenz in seiner Wirksamkeit erhöht oder komplett durch wirksame Promotoren ausgetauscht werden.

Dem Threonin-Aldolase-Gen gemäß SEQ ID No. 1 können des weiteren regulatorische Gen-Sequenzen bzw. Regulatorgene zugeordnet sein, die insbesondere die Threonin-Adolase-Gen-Aktivität erhöhen. So können dem Threonin-Aldolase-Gen beispielsweise sog. "enhancer" zugeordnet sein, die über eine verbesserte Wechselwirkung zwischen RNA-Polymerase und DNA eine erhöhte Threonin-Aldolase-Gen-Expression bewirken.

Dem Threonin-Aldolase-Gen gemäß SEQ ID No. 1 mit oder ohne vorgeschaltetem Promotor bzw. mit oder ohne Regulator-Gen können ein oder mehrere DNA-Sequenzen vor- und/oder nachgeschaltet sein, so daß das Gen in einer Gen-Struktur enthalten ist. Durch Klonierung des Threonin-Aldolase Gens sind Plasmide bzw. Vektoren erhältlich, die das Threonin-Aldolase-Gen oder eine Gen-Struktur der zuvor genannten Art enthalten und zur Transformation eines Riboflavin-Produzenten geeignet sind. Die vorliegende Erfindung umfasst dabei die Verwendung der Nukleotidsequenz gemäß SEQ ID No. 1 kodierend für ein Threonin-Aldolase-Gen oder meiner Genstruktur der zuvor genannten Art zur Herstellung eines erfindungsgemäßen genetisch veränderten Pilzes. Umfasst ist ferner die Verwendung des Vektors zur Herstellung des erfindungsgemäßen genetisch veränderten Pilzes. Die durch Transformation erhältlichen Zellen enthalten das Gen in replizierbarer Form, d.h. in zusätzlichen Kopien auf dem Chromosom, wobei die Genkopien durch homologe Rekombination an beliebigen Stellen des Genoms integriert werden.

Gegenstand der vorliegenden Erfindung ist auch ein Verfahren zur Herstellung eines Riboflavin produzierenden genetisch veränderten Pilzes der Gattung Ashbya durch Vorgehensweisen ausgewählt aus
a) einer Erhöhung der chromosomalen oder plasmidkodierten Genkopienzahl der Nukleinsäuresequenz gemäß SEQ ID No. 1, bevorzugt des Bereichs von Nukleotid 1 bis 1149 gemäß SEQ ID No. 1 und/oder
b) einem Austausch des Promotors von Nukleotid - 1231 bis -1 gemäß SEQ ID No. 1 mittels gentechnischer Methoden, wodurch eine verstärkte Initiation der Transkription erreicht wird.

Gegenstand der vorliegenden Erfindung ist ferner ein transformierter Pilz der Gattung Ashbya zur Herstellung von Riboflavin enthaltend in replizierbarer Form eine Nukleotidsequenz gemäß SEQ ID No. 1 der zuvor genannten Art oder eine Gen-Struktur wie zuvor beschrieben. Umfasst ist somit auch ein transformierter Pilz der Gattung Ashbya enthaltend einen Vektor der zuvor genannten Art.
Bevorzugt handelt es sich um Ashbya gossypii.

Die vorliegende Erfindung umfasst auch ein Verfahren zur Herstellung von Riboflavin, das sich dadurch auszeichnet, dass ein erfindungsgemäßer genetisch veränderter Pilz der Gattung Ashbya ohne Zusatz von Glycin zum Kulturmedium gezüchtet wird und das gebildete Riboflavin aufbereitet wird.

Das erfindungsgemäße Ziel einer teilweisen oder vollständigen intrazellulären Glycinbildung kann auch dadurch erreicht werden, daß genetisch veränderte Pilze hergestellt werden, bei denen der intrazelluläre Abbau des Glycins wenigstens teilweise blockiert ist. Derartige Mutationen können - wie bereits oben geschildert - nach klassischen Methoden durch physikalische oder chemische Mutagenese ungerichtet erzeugt werden, wie beispielsweise durch UV-Bestrahlung oder mutationsauslösende Chemikalien, oder gezielt mittels gentechnologischer Methoden.

Erfindungsgemäß kann das Ziel der erhöhten intrazellulären Glycinbildung vorzugsweise durch eine Veränderung des Gens der Serin-Hydroxymethyltransferase erreicht werden.

Erfindungsgemäß wurde überraschend festgestellt, daß hierzu Mutanten gehören, die gegen Glycin-Antimetaboliten resistent sind. Gegenstand der vorliegenden Erfindung ist auch ein genetisch veränderter ein- oder mehrzelliger Pilz, bei dem der intrazelluläre Abbau des Glycins durch die Hemmung der Serzinhydroxymethyltransferase-Aktivität durch Selektion von Mutanten, die gegen Glycin-Antimetaboliten resistent sind, gehemmt ist. Vorzugsweise handelt es sich um einen genetisch veränderten Pilz der Gattung Ashbya der gegenüber Alpha-Aminomethylphosphonsäure oder Alpha-Aminosulphonsäure resistent ist.

Ebenso kann dies z. B. durch die Selektion von Mutanten, die gegen das Threoninstrukturanalogon β-Hydroxy-Novalin und/oder an der Threonin- und/oder Lysin-Analoga resistent sind, erreicht werden.
Demgemäß können erfindungsgemäß einsetzbare Mutanten auch durch entsprechende Selektion hergestellt werden. Die Herstellung solcher resistenten genetisch veränderten ein- oder mehrzelligen Pilze läßt sich daher mit klassischen Screening-Methoden erreichen, wie sie in der Mikrobiologie allgemein üblich sind.

Zusammenfassend ist festzustellen, daß die erfindungsgemäße Aufgabe vorzugsweise durch Erhöhung der intrazellulären Synthese des Glycins, wenigstens teilweise Blockierung des Abbaus des Glycins, gelöst werden Kann.

Eine zusätzliche Steigerung der Riboflavinbildung läßt sich durch Zugabe von Glycin zum Nährmedium erzielen. In einer Variante des erfindungsgemäßen Verfahrens zur Herstellung von Riboflavin wird ein genetisch veränderter Pilz der zuvor genannten Art mit Zusatz von Glycin zum Kulturmedium gezüchtet. Das gebildete Riboflavin wird anschließend aufbereitet.

Bei den erfindungsgemäß erhaltenen genetisch veränderten Pilzen handelt es sich um transformierte Zellen von Pilzen, der Gattung Ashbya. Hierbei ist die Spezies Ashbya gossypii besonders bevorzugt.

Die vorliegende Erfindung umfasst somit auch die Verwendung eines erfindungsgemäßen genetisch veränderten Pilzes der Gattung Ashbya zur Herstellung von Riboflavin.

Im folgenden wird die Erfindung näher anhand von Beispielen erläutert, ohne daß damit eine Begrenzung auf den Gegenstand der Beispiele verbunden sein soll:

### Beispiel 1

### -Selektionierung einer gegen Alpha-Aminomethylphosphonsäure (AMPS) resistenten Mutante.

Mittels UV-Licht wurden Sporen von Ashbya gossypii mutagenisiert. Diese wurden auf mit 70 mM Alpha-Aminomethylphosphonsäure versetzte Platten gegeben. Die Hemmung der Riboflavin-Bildung ist dadurch erkennbar, daß der Pilz ohne Hemmstoff gelbe und mit Hemmstoff weiße Kolonien bildet. Demgemäß wurden die gelben, d.h. hemmstoffresistenten Organismen vereinzelt. Auf diese Art wurde u.a. der resistente Stamm AMPS-NM-01 erhalten.

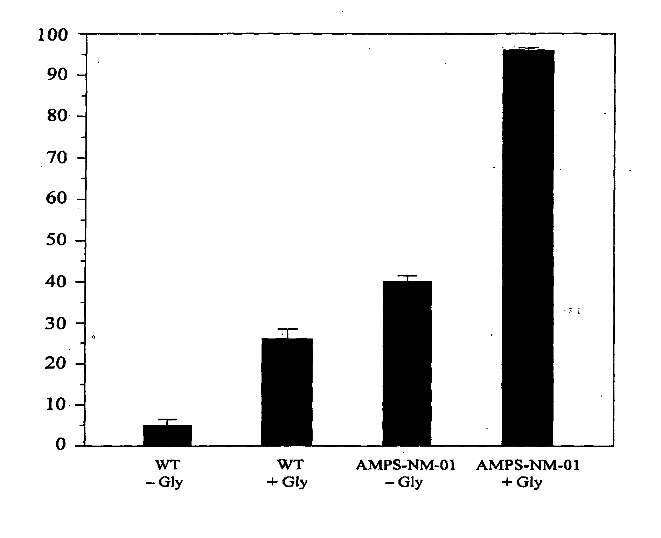

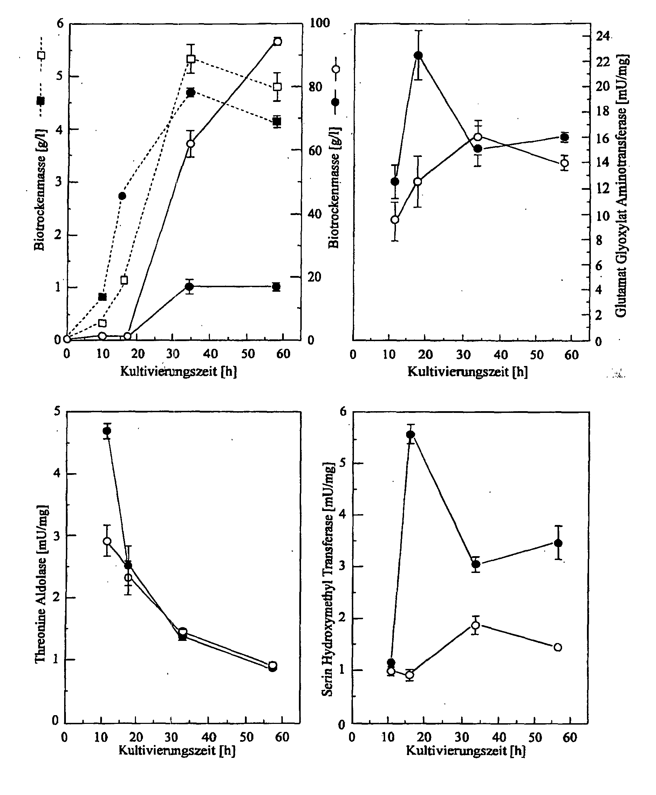

Versuche auf Platten mit 200 mM AMPS zeigten, daß der Stamm immer noch eine gelbe Koloniefarbe im Gegensatz zum Ausgangsstamm aufwies, der völlig weiß blieb. In Submerskultur zeigte die Mutante ohne Glycin die gleiche Riboflavin-Bildung wie der Wildtyp mit Glycin (vgl. Figur 1).
Untersuchungen der spezifischen Enzymaktivitäten von Wildtyp und Mutante ergaben eine auf 50% reduzierte Aktivität der Serin Hydroxymethyltransferase (Fig. 7). Da durch Fütterung von 13C-markiertem Threonin gezeigt werden konnte, daß eine Serinbildung aus Glycin erfolgt (Tabelle1), die vermutlich durch Serin Hydroxymethyltransferase katalysiert wird, kann die erhöhte Riboflavinbildung durch eine Reduzierung des Abflusses von Glycin zu Serin erklärt werden.

Das gemäß Tabelle 1 verwendete Minimalmedium setzt sich wie folgt zusammen:

| Lösung A: | KH₂PO₄ | | 200 | | g/l | pH6,7 mit KOH |
|---|---|---|---|---|---|---|
| (100 fach) | | | | | | |

| Lösung B: | NH₄Cl | 15 | | g/l | | |
|---|---|---|---|---|---|---|
| (10 fach) | Asparagin | | 5 | | g/l | |
| | NaCl | | 2 | | g/l | |
| | MgSO₄ x 7H₂O | | 4 | | g/l | |
| | MnSO₄ x H₂O | | 0,5 | | g/l | |
| | CaCl₂ x 2 H₂O | | 0,4 | | g/l | |
| | myo-Inositol | | 1,0 | | g/l | |
| | Nicotinsäureamid | | 2,5 | | g/l | |
| | Hefeextrakt | | 2 | | g/l | |

| C-Quelle: | Glucose oder Sojaöl | | 2,5 | | g/l | |
|---|---|---|---|---|---|---|
| | | | | | | |

Zur Herstellung des Mediums wurde einfach konzentrierte Lösung B mit der C-Quelle versetzt und durch Autoklavieren sterilisiert. Nach Abkühlung des Mediums wurde 1/100 Volumen getrennt autoklavierte Lösung A zugegeben.

### Beispiel 2

### Isolierung des GLY1-Gens aus Ashbya gossypii

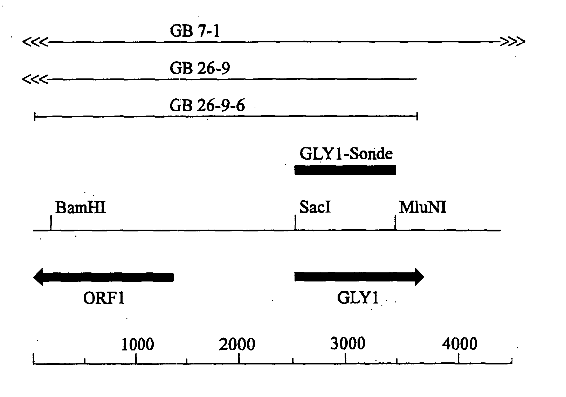

Zur Isolierung des Gens für die Threonin-Aldolase wurde die glycinauxotrophe Mutante von Saccharomyces cerevisiae YM 13F (SHM1 : : HIS3 shm2 : : LEU2 gly1 : : URA3) nach Selektion auf Fluororotsäureresistenz mit einer Genbank von Ashbya gossypii transformiert. Die Genbank bestand aus mit Sau3A partiell verdauter genomischer DNA, von der über Dichtegradientenzentrifugation 8-16 kb große Fragmente isoliert und in den mit BamH1 geschnitten Vektor Yep352 ligiert worden waren. Zuerst wurden die Transformanden auf Uracilprototrophie selektioniert. Im zweiten Schritt erfolgte nach Replikaplattierung eine Selektion auf Glycinprototrophie. Von etwa 70.000 Uracilprototophen Klonen konnten 25 Glycinprotrophe Klone isoliert werden. Kurierung der Transformanden sowie Retransformation mit den isolierten Plasmiden zeigte, daß die Komplementation plasmidkodiert war. Während im Glycinauxotrophen Saccharomyces-Stamm keine Threonin-Aldolase-Aktivität meßbar war (<0,1 mU/mg Protein), konnte in den mit den isolierten Genbankplasmiden transformierten Stämmen eine deutliche Enzymaktivität gemessen werden (25 mU/mg Protein). Ein subkloniertes 3,7 kb Hind III-Fragment, das Komplementation zeigte, wurde sequenziert (Figur 2). Es wurde ein dem GLY1 aus Saccharomyces cerevisiae homologes Gen, das für eine Threonin-Aldolase kodiert gefunden.

### Beispiel 3

### Überexpression des GLY1-Gens in Ashbya gossypii

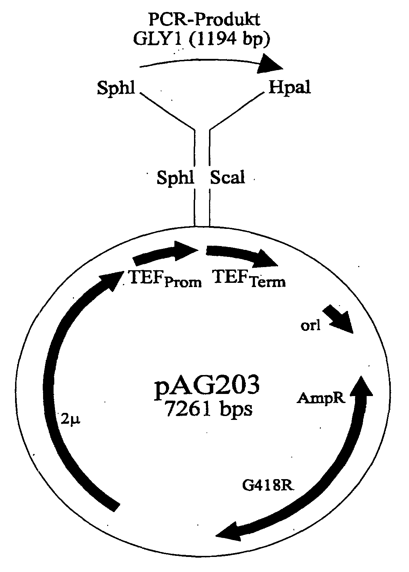

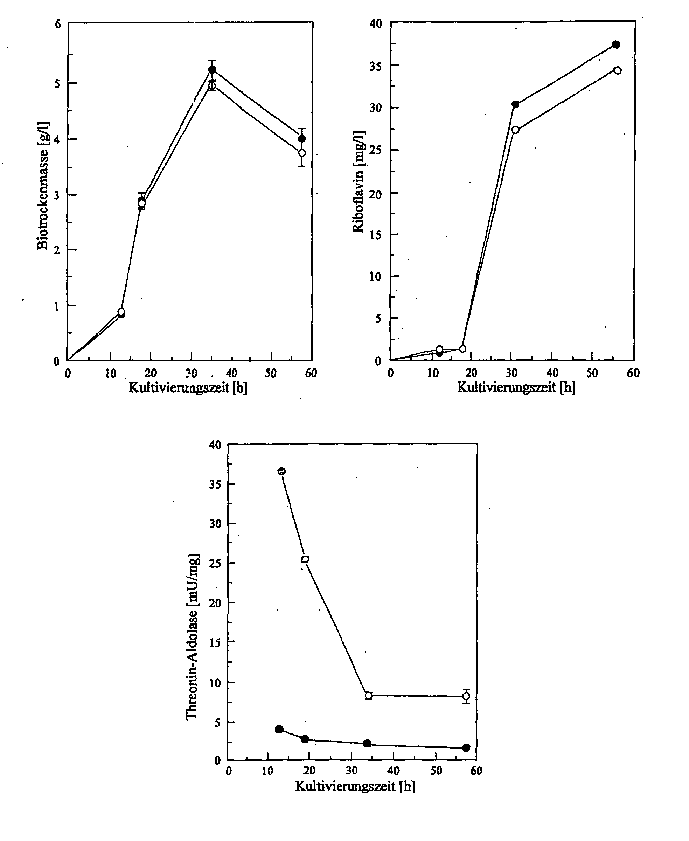

Zur Überexpression des GLY1-Gens wurde es in den Expressionsvektor pAG203 kloniert (vgl. WO9200379). In diesem Plasmid steht das Gen unter Kontrolle des TEF-Promotors und des TEF-Terminators (Figur 3). Als Selektionsmarker in Ashbya gossypii funktioniert ein G418 Resistenzgen. Nach Transformation von Ashbya gossypii mit diesem Plasmid und anschließender Isolierung von Einsporenklonen, weil die Sporen einkernig und haploid sind, wurde die Aktivität der Threonin-Aldolase im Rohextrakt gemessen. Im Vergleich zu einem mit dem leeren Plasmid pAG203 transformierten Stamm konnten bei A.g.p.AG203GLY1 sowohl bei Wachstum auf Glukose als auch bei Wachstum auf Sojaöl eine mindestens 10-fache Überexpression gemessen werden (Figur 4).

### Beispiel 4

### Steigerung der Riboflavin-Bildung durch GLY1-Überexpression und Fütterung von Threonin

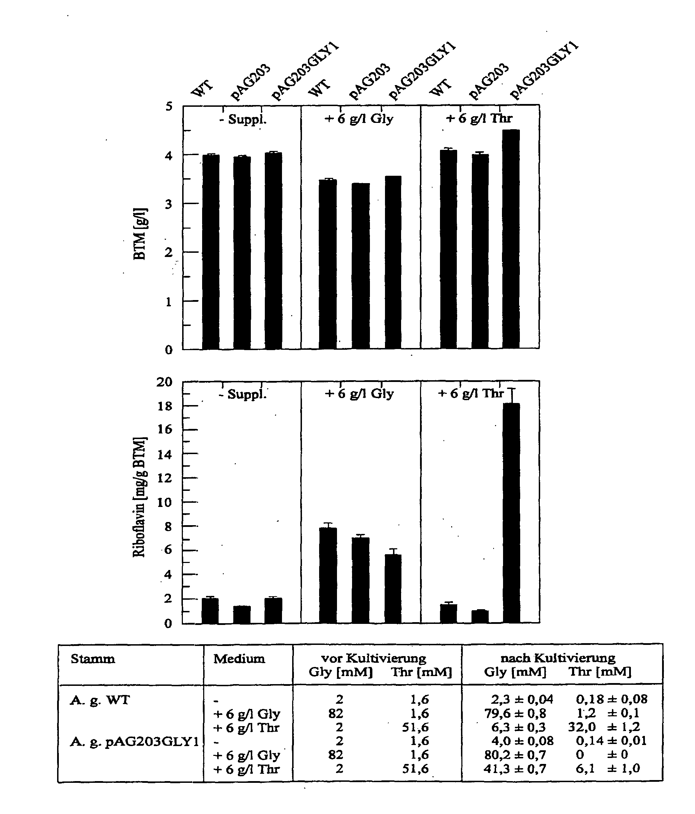

Um zu prüfen, ob das in der Zelle gebildete Threonin die Glycinbildung durch die überexprimierte Threonin-Aldolase limitiert, wurde Threonin ins Medium gegeben. Bei Zugabe von 6 Gramm pro Liter Threonin wurde bei Wachstum auf Glukose als Kohlenstoffquelle von A.g.pAG203GLY1 etwa doppelt soviel Riboflavin gebildet als bei Zugabe von 6 Gramm pro Liter Glycin (Figur 5). Der Test mit einem Wildtyp und einem mit dem Leerplasmid transformierten Kontrollstamm zeigten diesen Effekt nicht. Die Analyse der Aminosäuren im Medium ergab, daß beim GLY1-Überexprimierer nur noch etwa 6 mM der gefütterten 52 mM Threonin übrig waren und überraschenderweise die Glycinkonzentration von 2 mM auf 42 mM zugenommen hatte. Diese Ergebnisse zeigten eine Limitierung der Glycinbildung durch Threonin, die Funktionsfähigkeit der überexprimierten Threonin-Aldolase, eine bessere Wirksamkeit intrazellulär gebildeten Glycins im Vergleich zu extrazellulär gefüttertem und den massiven Export von Glycin durch die Pilzzellen.

### Beispiel 5

### Hemmung des Glycinexports

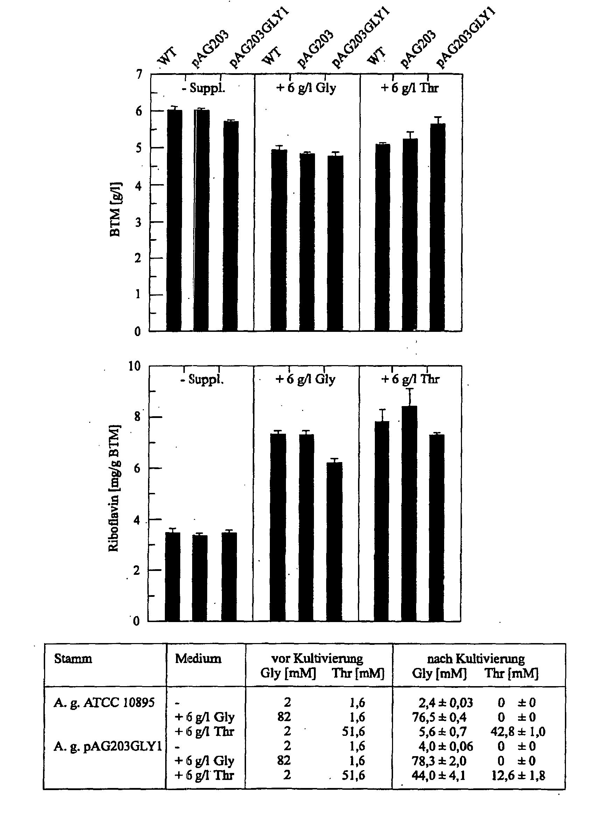

Wurde der Threoninaldolase überexprimierende Stamm A. g.pAG203GLY1 auf Sojaöl anstatt wie in Beispiel 4 auf Glucose gezüchtet, so ergab sich keine Steigerung der Riboflavinbildung bei Fütterung von Threonin, die die mit Glycin übertraf (Fig. 6). Die Analyse des Mediums zeigte aber, daß das Threonin bis auf etwa 13 mM abgebaut wurde. Eine Limitierung im Threonin kann also nicht vorgelegen haben. Gleichzeitig wurde gefunden, daß das extrazelluläre Glycin von 2 auf etwa 44 mM zugenommen hatte. Es war also alles gebildete Glycin vom Pilz in das Medium exportiert worden. Dieser Export ließ sich durch Vorlage von Glycin im Medium hemmen, was sich dann bei gleicher Threoninaufnahme in einer deutlich erhöhten Riboflavinbildung auswirkte (Tabelle 2). Um auszuschließen, daß nur das vorgelegte Glycin für die gesteigerte Produktion verantwortlich ist, wurde in einer Kontrolle soviel Glycin vorgelegt, wie im Experiment mit Glycin und Threonin letztlich an Glycin entstand. Dieser Befund unterstreicht, daß intrazellulär gebildetes Glycin viel wirksamer ist als extrazellulär zugegebenes.

### Beispiel 6

### Steigerung der Riboflavin-Bildung durch Selektion von β-Hydroxy-Norvalin resistenten Mutanten.

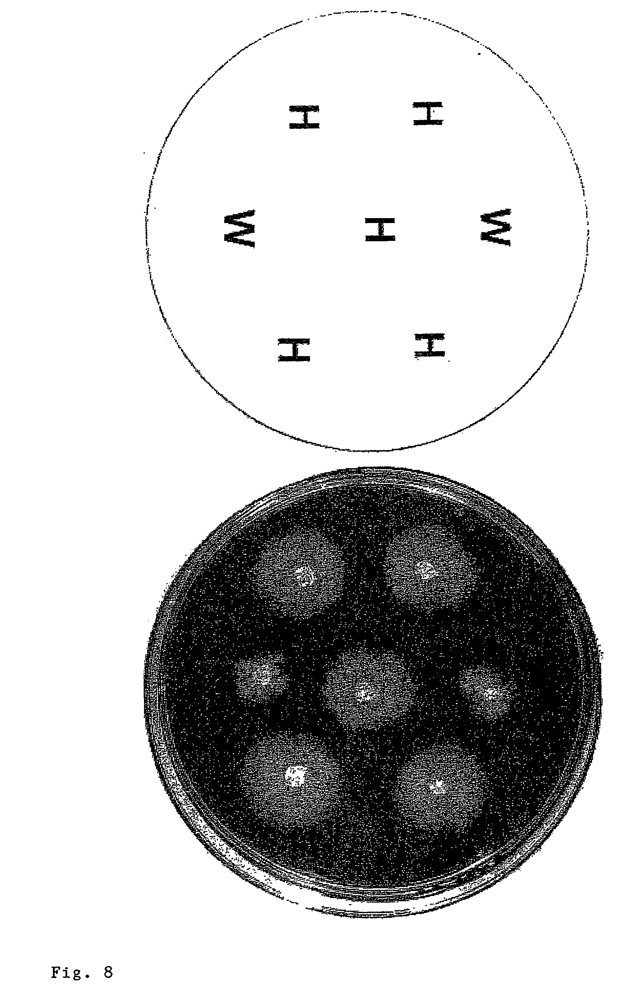

Da nicht die Threoninumsetzung zu Glycin, sondern die Threoninsynthese als erstes die Glycinbildung limitierte, wurde mit dem Threoninanalogon β-Hydroxy-Norvalin nach resistenten Mutanten gesucht. Auf Agarplatten mit Minimalmedium, das 2,5 mM β-Hydroxy-Norvalin enthielt, war das radiäre Wachstum deutlich gehemmt. An den Kolonierändern bildeten sich spontan Mutanten, die besser wuchsen. Durch Isolierung von Sporen und erneute Selektion ließen sich stabile Mutanten erzeugen, die auf dem Minimalmedium β-Hydroxy-Norvalin deutlich besser wuchsen als die Elternstämme (Fig. 8). Eine Untersuchung der Riboflavinbildung zeigte eine deutliche Steigerung der Produktivität. So bildete der Stamm HNV-TB-25 in Minimalmedium mit Sojaöl 41 ± 11 mg/l Riboflavin während sein Elternstamm nur 18 ± 3 mg/l produzierte. Auch der Abkömmling HNV-TB-29 zeigt mit 116 ± 4 mg/l eine deutliche Steigerung gegenüber seinem Ursprungsstamm Ita-GS-01, der nur 62 ± 10 mg/l bildete.

**Tabelle 1:**

| ¹³C-Anreicherung in den C-Atomen von Serin, Threonin und Glycin nach Wachstum von A. gossypii ATC10895 auf den angegebenen Medien und anschließender Totalhydrolyse der resultierenden Biomasse. (MM: Minimalmedium; HE: Hefeextrakt; YNB: Yeast Nitrogen Base; n. b.: nicht bestimmt) | | | |
|---|---|---|---|
| Medium | | MM + 0,2 g/l HE + 1 g/l Ethanol + 2,7 mg/l ¹³C₂-Serin | MM + 0,2 g/l YNB + 1 g/l Ethanol + 2,6 mg/l ¹³C₁-Threonin |
| Serin | C₁ | 1,1 | 4,9 |
| | C₂ | 5,9 | 1,1 |
| | C₃ | 1,1 | 1,1 |
| Threonin | C₁ | n.b. | 39,0 |
| | C₂ | | 1,1 |
| | C₃ | | 1,1 |
| | C₄ | | 1,1 |
| Glycin | C₁ | 1,1 | 7,1 |
| | C₂ | 4,3 | 1,1 |

**Tabelle 2:**

| Einfluß von Threonin- und Glycin-Supplementation auf die Riboflavinbildung bei gleichzeitiger Überexpression von GLY1 | | | | | |
|---|---|---|---|---|---|
| Stamm | Kohlenstoffquelle | t = 0 Supplement | t = 72 h Riboflavin [mg/l] | t = 72 h Gly [mM] | t = 72 h Thr [mM] |
| WT | Sojaöl | 80 mM Gly | | 80 ± 2 | |
| | | 50 mM Thr | 22 ± 1 | | 42 ± 0 |
| | | 130 mM Gly | 18 ± 3 | 129 ± 2 | n. d. |
| | Glucose | 80 mM Gly | | 80 ± 0 | |
| | | 50 mM Thr | 5 ± 1 | | 35 ± 0 |
| | | 130 mM Gly | 7 ± 1 | 126 ± 2 | n. d. |
| Ag pAG 203 GLY1 | Sojaöl | 80 mM Gly | | 117 ± 2 | |
| | | 50 mM Thr | 31 ± 0 | | 11 ± 1 |
| | | 130 mM Gly | 20 ± 3 | 129 ± 1 | n. d. |
| | Glucose | 80 mM Gly | | 113 ± 2 | |
| | | 50 mM Thr | 40 ± 1 | | 12 ± 0,7 |
| | | 130 mM Gly | 9 ± 1 | 129 ± 3 | n. d. |

| | | | | | |
|---|---|---|---|---|---|
| n. d. = nicht detektierbar | | | | | |

### Erläuterungen zu den Figuren

- **Figur 1:**: Riboflavinbildung der Ashbya gossypii-Stämme ATCC 10895 (Wildtyp, WT) und der AMPS-resistenten Mutante AMPS-MN-01 mit oder ohne 6 g/l Glycin nach Wachstum auf Vollmedium mit 10 g/l Sojaöl als Kohlenstoffquelle. Die Meßwerte stammen aus drei unabhängigen Experimenten.
- **Figur 2a:**: Gly 1-Lokus im Genom von Ashbya gossypii. Die Klone GB 7-1 und GB 26-9 sowie der 3,7 kb HindIII-Subklon GB-26-9-6 komplementieren die S. cerevisiae-Mutante. GB-26-9-6 wurde ganz, GB 7-1 zur Vervollständigung des C-Terminus von GLY1 sequenziert.
- **Figur 2b:**: Nukleotidsequenz und davon abgeleitete Aminosäuresequenz des A. gossypii GLY1-Gens sowie flankierende Nukleotidsequenz.
- **Figur 3:**: Schematische Darstellung der Konstruktion des Vektores pAG203GLY1 zur Überexpression des GLY1-Genes in A. gossypii.
- **Figur 4:**: Vergleich von Ashbya gossypii Wildtyp (gefüllte Symbole) und A.g.pAG203GLY1 (leere Symbole) bezüglich Wachstum, Riboflavinbildung und spezifischer Aktivität der Threonin-Aldolase während einer Kultivierung auf Vollmedium mit 10 g/l Sojaöl.
- **Figur 5:**: Wachstum und Riboflavinbildung der Ashbya gossypii-Stämme ATCC 10895 (Wildtyp), pAG203 und pAG203GLY1 bei Kultivierung auf HA-Vollmedium mit 10 g/l Glucose als C-Quelle undGlycin- bzw. Threoninsupplementierung. Die Tabelle zeigt die Glycin- und Threoninkonzentrationen im Medium jeweils vor und nach der Kultivierung. Die angegebenen Mittelwerte und Standardabweichungen sind das Ergebnis dreier unabhängiger Experimente.
- **Figur 6:**: Wachstum und Riboflavinbildung der Ashbya gossypii-Stämme ATCC 10895 (Wildtyp), pAG203 und pAG203GLY1 bei Kultivierung auf Vollmedium mit 10 g/l Glucose als C-Quelle undGlycin- bzw. Threoninsupplementierung. Die Tabelle zeigt die Glycin- und Threoninkonzentrationen im Medium jeweils vor und nach der Kultivierung. Die angegebenen Mittelwerte und Standardabweichungen sind das Ergebnis dreier unabhängiger Experimente.
- **Figur 7:**: Vergleich von Ashbya gossypii Wildtyp (gefüllte Symbole) und AMPS-resistenter Mutante AMPS-NM-01 bezüglich Wachstum, Riboflavinbildung sowie spezifische Aktivitäten der Threonin Aldolase, Serin Hydroxymethyltransferase und Glutamat Glyoxylat Aminotransferase während der Kultivierung auf Vollmedium mit 10 g/l Sojaöl. Die Meßwerte stammen aus drei unabhängigen Experimenten.
- **Figur 8:**: Wirkung von β-Hydroxy-Norvalin auf Ashbya gossypii Wachstum von Wildtyp (W) und HNV-TB-25 (H) auf einer Agarplatte mit Minimalmedium, das 2,5 g/l Glucose und 2,5 mM βHydroxynorvalin enthält.

### SEQUENZPROTOKOLL

(1) ALLGEMEINE ANGABEN:
   (i) ANMELDER:
      (A) NAME: Forschungszentrum Juelich GmbH
      (B) STRASSE: Leo-Brandt-Str.
      (C) ORT: Juelich
      (E) LAND: Germany
      (F) POSTLEITZAHL: D-52428
      (G) TELEFON: 02461 61-2843
      (H) TELEFAX: 02461 61-2710

      (A) NAME: BASF Aktiengesellschaft
      (B) STRASSE: ZDX/A C6
      (C) ORT: Ludwigshafen
      (E) LAND: Germany
      (F) POSTLEITZAHL: D-67056
   (ii) BEZEICHNUNG DER ERFINDUNG: Ein- oder Mehrzellige Organismen zur Herstellung von Riboflavin
   (iii) ANZAHL DER SEQUENZEN: 2
   (iv) COMPUTER-LESBARE FASSUNG:
      (A) DATENTR□GER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) L□NGE: 2744 Basenpaare
      (B) ART: Nucleotid
      (C). STRANGFORM: Doppelstrang
      (D) TOPOLOGIE: nicht bekannt
   (ii) ART DES MOLEK 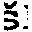 sLS: Genom-DNA
   (iv) ANTISENSE: NEIN
   (v) ART DES FRAGMENTS: N-Terminus
   (vi) URSPR 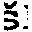 NLICHE HERKUNFT:
      (A) ORGANISMUS: Ashbya gossypii
   (ix) MERKMAL:
      (A) NAME/SCHL 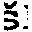 SSEL: CDS
      (B) LAGE:1232..2377
      (C) ART DER ERMITTLUNG: experimentell
      (D) SONSTIGE ANGABEN:/codon_start= 1232
         /product= "Threonin-Aldolase"
         /evidence= EXPERIMENTAL
         /number= 1
   (xi) SEQUENZBESCHREIBUNG: SEQ ID NO: 1: TTGCCATTAA TGACCGGGAG CCTGAAGGTG TGTGATGAAC AAGCCAGTCT TCCCCGCGCG 60 
TCGCCAACTG CTCGTCATAT AATCCCGGAA AAGCTCGCAT TAGGTGAAAT TTTTCTTAGG 120

AATTACATCT GCTACTGACA AAACTAAGTA AAAGCTCCGA TAGGTAGCCG TGCTGCCGAG 180

CACCTGCCTA ATACACGCAG GCGCCATACA CTATTTAAGC ACAATGTTAT CGCCCCGCAG 240

CTTGAGGTAT TCCTGGTCGA TGCCAGGTGT CATAGGCTTG ATCACCAGCG AGTAGACCTC 300

ACTATTGTAG AAGCGCAGCC CGTTGCTGGG GGACTTGTAG CGCGCCTTGA GCCCCGTGAT 360

GTCGCAGTAG CGTTTCACGG GATACTGCGA TGGTGGCGCC TGAATGTTGA AGTATGTCAG 420

CTTCGTGCGC CCTGCGTCAC GCCCGGCTTC CGACTGTGCC TCTGTCGTGA GCCGTTTCCA 480

CTCGTCTGTC AGAAGCTGAC GTGTCGGCTT GTGGCGGCGC GTGGGTTTCT TCCACGTGGG 540

CGACTTGAAG TCGCTACGAC TGGTATCATT ACGTGCTGCA ATCGCTCGGA GGTTCTCCAT 600

CTGGGGTCCA CGGTCGCTCG TTGATCTGTC TATCTCGAAA TCCCTGCCCA GATGTACTCC 660

CATGTTATCA CGTGACCACA CGCCGTTTTC GTGTGTAGTG ATGCAGATGG TTCTAGAGCA 720

TCACGTGGCT TACATAGCTT TGTTACATAA TCGATTTTCC GCAGGAGCGT TACGTCCAAC 780

GGTCGTTCTG TGCCAAAAGC AACAACTGAG CGTCAGGCGG CCGTCTCCCC AGACACGCTC 840

CGCCCCAAAC TGAGCTCCAC GCGGCCTTCT GTCCGAGTTA AGTTCCTCCC CGCTCGTCAG 900

CACGGGGTCT TTCGTCGCCT ATCCTCCTGC AGCGTTCGCT ACTGCAGATC GTGAGCAGTG 960

GCACCCGCGA CCAAAAAAAG AAATTATGTT CCTTACGCAA GGAATATGCC TCGCGCCATG 1020

CCATCGCAAA GAGTGATGCC GCAGAGGTTG CTTCTGCGAG GCAACTCCTG GGCAATAGGG 1080

TGGAAAATTC AGCTTGGGCT TATATAAAAG AAACCGTTCG AGCTCGTCGG AGCCAGGTGG 1140

AAAATTTTTC GTAACGTAGG TAGAGGTTAT AGTTAGCGTC AGTCTCTTTT CTGCCAAGCT 1200

GCTACAGTTG ACTACAAGTA ACAAACCCAG G ATG AAT CAG GAT ATG GAA CTA 1252
Met Asn Gln Asp Met Glu Leu
1               5

CCA GAG GCG TAC ACG TCG GCT TCG AAC GAC TTC CGT TCG GAC ACG TTC 1300
Pro Glu Ala Tyr Thr Ser Ala Ser Asn Asp Phe Arg Ser Asp Thr Phe
        10                  15                  20

ACC ACT CCA ACG CGC GAA ATG ATC GAG GCT GCG CTA ACG GCG ACC ATC 1348
Thr Thr Pro Thr Arg Glu Met Ile Glu Ala Ala Leu Thr Ala Thr Ile
    25                  30                  35

GGT GAC GCC GTC TAC CAA GAG GAC ATC GAC ACG TTG AAG CTA GAA CAG 1396
Gly Asp Ala Val Tyr Gln Glu Asp Ile Asp Thr Leu Lys Leu Glu Gln
40                  45                  50                  55

CAC GTC GCC AAG CTG GCC GGC ATG GAG GCC GGT ATG TTC TGC GTA TCT 1444
His Val Ala Lys Leu Ala Gly Met Glu Ala Gly Met Phe Cys Val Ser
                60                  65                  70

GGT ACT TTG TCC AAC CAG ATT GCT TTG CGG ACC CAC CTA ACT CAG CCA 1492
Gly Thr Leu Ser Asn Gln Ile Ala Leu Arg Thr His Leu Thr Gln Pro
            75                  80                  85
 
CCA TAT TCG ATT CTT TGC GAC TAC CGT GCG CAT GTG TAC ACG CAC GAG 1540
Pro Tyr Ser Ile Leu Cys Asp Tyr Arg Ala His Val Tyr Thr His Glu
        90                  95                  100

GCT GCG GGG TTG GCA ATT TTG TCC CAG GCC ATG GTG ACA CCT GTC ATT 1588
Ala Ala Gly Leu Ala Ile Leu Ser Gln Ala Met Val Thr Pro Val Ile
    105                 110                 115

CCT TCC AAC GGC AAC TAC TTG ACT TTG GAA GAC ATC AAG AAG CAC TAC 1636
Pro Ser Asn Gly Asn Tyr Leu Thr Leu Glu Asp Ile Lys Lys His Tyr
120                 125                 130                 135

ATT CCT GAT GAT GGC GAC ATC CAC GGT GCT CCA ACA AAG GTT ATC TCG 1684
Ile Pro Asp Asp Gly Asp Ile His Gly Ala Pro Thr Lys Val Ile Ser
                140                 145                 150

TTG GAA AAC ACC TTG CAC GGT ATC ATT CAC CCA CTA GAG GAG CTT GTT 1732
Leu Glu Asn Thr Leu His Gly Ile Ile His Pro Leu Glu Glu Leu Val
            155                 160                 165

CGG ATC AAG GCT TGG TGT ATG GAG AAC GAC CTC AGA CTA CAC TGC GAT 1780
Arg Ile Lys Ala Trp Cys Met Glu Asn Asp Leu Arg Leu His Cys Asp
        170                 175                 180

GGT GCG AGA ATC TGG AAC GCG TCC GCA GAA TCC GGT GTG CCT CTA AAA 1828
Gly Ala Arg Ile Trp Asn Ala Ser Ala Glu Ser Gly Val Pro Leu Lys
    185                 190                 195

CAG TAC GGA GAG CTA TTC GAC TCC ATT TCC ATC TGC TTG TCC AAG TCC 1876
Gln Tyr Gly Glu Leu Phe Asp Ser Ile Ser Ile Cys Leu Ser Lys Ser
200                 205                 210                 215

ATG GGT GCC CCA ATG GGC TCC ATT CTC GTC GGG TCG CAC AAG TTC ATA 1924
Met Gly Ala Pro Met Gly Ser Ile Leu Val Gly Ser His Lys Phe Ile
                220                 225                 230

AAG AAG GCG AAC CAC TTC AGA AAG CAG CAA GGT GGT GGT GTC AGA CAG 1972
Lys Lys Ala Asn His Phe Arg Lys Gln Gln Gly Gly Gly Val Arg Gln
            235                 240                 245

TCT GGT ATG ATG TGC AAG ATG GCG ATG GTG GCT ATC CAG GGT GAC TGG 2020
Ser Gly Met Met Cys Lys Met Ala Met Val Ala Ile Gln Gly Asp Trp
        250                 255                 260

AAG GGC AAG ATG AGG CGT TCG CAC AGA ATG GCT CAC GAG CTG GCC AGA 2068
Lys Gly Lys Met Arg Arg Ser His Arg Met Ala His Glu Leu Ala Arg
    265                 270                 275

TTT TGC GCA GAG CAC GGC ATC CCA TTG GAG TCG CCT GCT GAC ACC AAC 2116
Phe Cys Ala Glu His Gly Ile Pro Leu Glu Ser Pro Ala Asp Thr Asn
280                 285                 290                 295

TTT GTC TTT TTG GAC TTG CAG AAG AGC AAG ATG AAC CCT GAC GTG CTC 2164
Phe Val Phe Leu Asp Leu Gln Lys Ser Lys Met Asn Pro Asp Val Leu
                300                 305                 310

GTC AAG AAG AGT TTG AAG TAC GGC TGC AAG CTA ATG GGC GGG CGT GTC 2212
Val Lys Lys Ser Leu Lys Tyr Gly Cys Lys Leu Met Gly Gly Arg Val
            315                 320                 325

TCC TTC CAC TAC CAG ATA TCT GAG GAG TCC CTT GAG AAG ATC AAG CAG 2260
Ser Phe His Tyr Gln Ile Ser Glu Glu Ser Leu Glu Lys Ile Lys Gln
        330                 335                 340
 
GCC ATC CTA GAG GCG TTC GAG TAC TCG AAG AAG AAC CCT TAC GAT GAA 2308
Ala Ile Leu Glu Ala Phe Glu Tyr Ser Lys Lys Asn Pro Tyr Asp Glu
    345                 350                 355

AAC GGC CCC ACG AAG ATC TAC AGA AGT GAG TCC GCT GAC GCT GTG GGT 2356
Asn Gly Pro Thr Lys Ile Tyr Arg Ser Glu Ser Ala Asp Ala Val Gly
360                 365                 370                 375

GAG ATC AAG ACC TAC AAG TAT TAAGGATTT CGATGATGAC ATGAAAAATT 2407
Glu Ile Lys Thr Tyr Lys Tyr
                380

ACATATTGGC ACGGCATAGG CATTGGGTAA TATTAAGCAT ATGGTTGAGA TGAATTACTG 2467

TTCGGGTACC GGTATTTCCA AAGTGCTGTC GACTTTTGCA AGAGATGGCT ATGAATGGGG 2527

CACGCTCCAT CACCTCTCTG CGAGCCGGAC TCAGCATTAT ATCCATCTCA AAACCTAATA 2587

TCAAATGGGA TTGTGGTGCG CAGTACATGC GCAGTGCTGC ACATTTGAGG ATCAATGGGT 2647

TTTTCCAGGC ACTGCCTGGG TCACTCACCC TATTGCGGAG GGACTAGTAG CTCTACCATT 2707

CTGAGCTGAC TAAAATGTTT GATTCTTTTG GTACTTA 2744
(2) ANGABEN ZU SEQ ID NO: 2:
   (i) SEQUENZKENNZEICHEN:
      (A) L□NGE: 382 Aminos"uren
      (B) ART: Aminos"ure
      (D) TOPOLOGIE: linear
   (ii) ART DES MOLEK 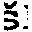 LS: Protein
   (xi) SEQUENZBESCHREIBUNG: SEQ ID NO: 2:

Met Asn Gln Asp Met Glu Leu Pro Glu Ala Tyr Thr Ser Ala Ser Asn
1               5                   10                  15

Asp Phe Arg Ser Asp Thr Phe Thr Thr Pro Thr Arg Glu Met Ile Glu
            20                  25                  30

Ala Ala Leu Thr Ala Thr Ile Gly Asp Ala Val Tyr Gln Glu Asp Ile
        35                  40                  45

Asp Thr Leu Lys Leu Glu Gln His Val Ala Lys Leu Ala Gly Met Glu
    50                  55                  60

Ala Gly Met Phe Cys Val Ser Gly Thr Leu Ser Asn Gln Ile Ala Leu
65                  70                  75                  80

Arg Thr His Leu Thr Gln Pro Pro Tyr Ser Ile Leu Cys Asp Tyr Arg
                85                  90                  95

Ala His Val Tyr Thr His Glu Ala Ala Gly Leu Ala Ile Leu Ser Gln
            100                 105                 110

Ala Met Val Thr Pro Val Ile Pro Ser Asn Gly Asn Tyr Leu Thr Leu
        115                 120                 125

Glu Asp Ile Lys Lys His Tyr Ile Pro Asp Asp Gly Asp Ile His Gly
    130                 135                 140

Ala Pro Thr Lys Val Ile Ser Leu Glu Asn Thr Leu His Gly Ile Ile
145                 150                 155                 160

His Pro Leu Glu Glu Leu Val Arg Ile Lys Ala Trp Cys Met Glu Asn
                165                 170                 175

Asp Leu Arg Leu His Cys Asp Gly Ala Arg Ile Trp Asn Ala Ser Ala
            180                 185                 190

Glu Ser Gly Val Pro Leu Lys Gln Tyr Gly Glu Leu Phe Asp Ser Ile
        195                 200                 205

Ser Ile Cys Leu Ser Lys Ser Met Gly Ala Pro Met Gly Ser Ile Leu
    210                 215                 220

Val Gly Ser His Lys Phe Ile Lys Lys Ala Asn His Phe Arg Lys Gln
225                 230                 235                 240

Gln Gly Gly Gly Val Arg Gln Ser Gly Met Met Cys Lys Met Ala Met
                245                 250                 255

Val Ala Ile Gln Gly Asp Trp Lys Gly Lys Met Arg Arg Ser His Arg
            260                 265                 270

Met Ala His Glu Leu Ala Arg Phe Cys Ala Glu His Gly Ile Pro Leu
        275                 280                 285

Glu Ser Pro Ala Asp Thr Asn Phe Val Phe Leu Asp Leu Gln Lys Ser
    290                 295                 300

Lys Met Asn Pro Asp Val Leu Val Lys Lys Ser Leu Lys Tyr Gly Cys
305                 310                 315                 320

Lys Leu Met Gly Gly Arg Val Ser Phe His Tyr Gln Ile Ser Glu Glu
                325                 330                 335

Ser Leu Glu Lys Ile Lys Gln Ala Ile Leu Glu Ala Phe Glu Tyr Ser
            340                 345                 350

Lys Lys Asn Pro Tyr Asp Glu Asn Gly Pro Thr Lys Ile Tyr Arg Ser
        355                 360                 365

Glu Ser Ala Asp Ala Val Gly Glu Ile Lys Thr Tyr Lys Tyr
    370                 375                 380

## Patentansprüche

1. Genetisch veränderter Pilz der Gattung Ashbya zur biotechnischen Herstellung von Riboflavin, **dadurch gekennzeichnet, daß** er via Transformation eine Nukleotidsequenz gemäß SEQ ID No. 1 kodierend für eine Threonin-Aldolase in replizierbarer Form mit einer Kopienzahl größer 1 aufweist und durch Expression besagter Nukleotidsequenz eine gegenüber seiner endogenen Threonin-Aldolase-Aktivität erhöhte katalytische Aktivität dieses Enzyms aufweist.

2. Genetisch veränderte Pilz nach Anspruch 1,
**dadurch gekennzeichnet, daß** seine Riboflavinsyntheseleistung ohne externe Zufuhr von Glycin mindestens gleich derjenigen eines Wildtyps der Species Ashbya ATCC10895 ist, der unter Standardbedingungen unter Zugabe von 6 g/l externen Glycin gezüchtet wird.

3. Genetisch veränderter Pilz nach einem der Ansprüche 1 oder 2,
**dadurch gekennzeichnet, daß** bei ihm der intrazelluläre Abbau des Glycins durch die Hemmung der Serinhydroxymethyltransferase-Aktivität durch Selektion von Mutanten, die gegen Glycin-Antimetaboliten resistent sind, gehemmt ist.

4. Genetisch veränderter Pilz nach Anspruch 3,
**dadurch gekennzeichnet, daß** er gegenüber Alpha-Aminomethylphosphonsäure oder Alpha-Aminosulfonsäure, β-Hydroxy-Norvalin und/oder andere Threonin- und/oder Lysin-Analogen resistent ist.

5. Genetisch veränderter Pilz nach einem der Ansprüche 1 bis 4,
**dadurch gekennzeichnet, daß** er Ashbya gossypii ist.

6. Threonin-Aldoläse mit einer für die in SEQ ID No. 2 angegebenen Aminosäuresequenz.

7. Nukleotidsequenz gemäß SEQ ID No. 1 kodierend für eine Threonin-Aldolase.

8. Nukleotidsequenz nach Anspruch 7, mit einer Kodierregion für ein Threonin-Aldolase-Gen von Nukleotid 1232 bis 2377 gem. SEQ ID No. 1.

9. Nukleotidsequenz nach einem der Ansprüche 7 oder 8 mit einem dem Aldolase-Gen vorgeschalteten Promotor von Nukleotid 1 bis 1231 gem. SEQ ID No. 1.

10. Gen-Struktur enthaltend ein Threonin-Aldolase-Gen mit einer Nukleotidsequenz nach einem der Ansprüche 7 bis 9 und diesem zugeordnete regulatorische Gensequenzen.

11. Vektor enthaltend ein Threonin-Aldolase-Gen mit einer Nukleotidsequenz nach einem der Ansprüche 7 bis 9 oder eine Gen-Struktur nach Anspruch 10.

12. Transformierter Pilz der Gattung Ashbya zur Herstellung von Riboflavin enthaltend in replizierbarer Form eine Nukleotidsequenz nach einem der Ansprüche 7 bis 9 oder eine Gen-Struktur nach Anspruch 10.

13. Transformierter Pilz nach Anspruch 12 enthaltend einen Vektor nach Anspruch 11.

14. Transformierter Pilz nach einem der Ansprüche 12 oder 13, der ein Pilz der Spezies Ashbya gossypii ist.

15. Verfahren zur Herstellung eines Riboflavin produzierenden genetisch veränderten Pilzes gemäß Anspruch 1 durch Vorgehensweisen ausgewählt aus
a) einer Erhöhung der chromosomalen oder plasmidkodierten Genkopienzahl der Nukleinsäuresequenz gemäß SEQ ID No. 1, bevorzugt des Bereichs von Nukleotid 1 bis 1149 gemäß SEQ ID No. 1 und/oder
b) einem Austausch des Promotors von Nukleotid -1231 bis -1 gemäß SEQ ID No. 1 mittels gentechnischer Methoden, wodurch eine verstärkte Initiation der Transkription erreicht wird.

16. Verfahren zur Herstellung von Riboflavin,
**dadurch gekennzeichnet, daß** ein genetisch veränderter Pilz gemäß einem der Ansprüche 1 bis 5 oder 12 bis 14 ohne Zusatz von Glycin zum Kulturmedium gezüchtet wird und das gebildete Riboflavin aufbereitet wird.

17. Verfahren nach Anspruch 16,
**dadurch gekennzeichnet, daß** der genetisch veränderter Pilz gemäß einem der Ansprüche 1 bis 5 oder 12 bis 14 mit Zusatz von Glycin zum Kulturmedium gezüchtet wird.

18. Verwendung des genetisch veränderten Pilzes nach einem der Ansprüche 1 bis 5 oder 12 bis 14 zur Herstellung von Riboflavin.

19. Verwendung der Nukleotidsequenz gemäß SEQ ID No. 1 kodierend für ein Threonin-Aldolase-Gen nach einem der Ansprüche 7 bis 9 oder der Gen-Struktur nach Anspruch 10 zur Herstellung eines genetisch veränderten Pilzes nach einem der Ansprüche 1 bis 5 oder 12 bis 14.

20. Verwendung des Vektors nach Anspruch 11 zur Herstellung eines genetisch veränderten Pilzes nach einem der Ansprüche 1 bis 5 oder 12 bis 14.

## Claims

1. A genetically modified fungus of the genus Ashbya for biotechnologically preparing riboflavin, which possesses, by way of transformation, in replicable form with a copy number greater than 1, a nucleotide sequence, as depicted in SEQ ID No. 1, encoding a threonine aldolase, and which exhibits, by expression of said nucleotide sequence, a catalytic activity of this enzyme which is greater than that of its endogenous threonine aldolase.

2. A genetically modified fungus according to claim 1, whose riboflavin synthesis capacity without glycine being supplied externally is at least the same as that of a wild type of the species Ashbya ATCC10895 which is grown under standard conditions in the added presence of 6 g of external glycine/l.

3. A genetically modified fungus according to claim 1 or 2,
wherein the intracellular breakdown of glycine is inhibited by means of inhibiting serine hydroxymethyltransferase activity by selecting mutants which are resistant to glycine antimetabolites.

4. A genetically modified fungus according to claim 3, which is resistant to alpha-aminomethylphosphonic acid or alpha-aminosulfonic acid or β-hydroxynorvaline and/or other threonine analogs and/or lysine analogs.

5. A genetically modified fungus according to one of claims 1 to 4,
which is Ashbya gossypii.

6. A threonine aldolase having an amino acid sequence as specified in SEQ ID No. 2.

7. A nucleotide sequence as depicted in SEQ ID No. 1 encoding a threonine aldolase.

8. The nucleotide sequence according to claim 7, having a region from nucleotide 1232 to 2377, as depicted in SEQ ID No. 1, encoding a threonine aldolase gene.

9. The nucleotide sequence according to claim 7 or 8 having a promoter which is located upstream of the aldolase gene, from nucleotide 1 to 1231 as depicted in SEQ ID No. 1.

10. A gene structure containing a threonine aldolase gene having a nucleotide sequence according to one of claims 7 to 9 and regulatory gene sequences which are assigned thereto.

11. A vector containing a threonine aldolase gene having a nucleotide sequence according to one of claims 7 to 9 or a gene structure according to claim 10.

12. A transformed fungus of the genus Ashbya for preparing riboflavin, containing, in replicatable form, a nucleotide sequence according to one of claims 7 to 9 or a gene structure according to claim 10.

13. The transformed fungus according to claim 12 containing a vector according to claim 11.

14. The transformed fungus according to claim 12 or 13 which is a fungus of the species Ashbya gossypii.

15. A method for preparing a riboflavin-producing genetically modified fungus according to claim 1 by means of procedures selected from
a) an increase in the chromosomal or plasmid-encoded gene copy number of the nucleic acid sequence as depicted in SEQ ID No. 1, preferably of the region from nucleotide 1 to 1149 as depicted in SEQ ID No. 1, and/or
b) a replacement of the promoter from nucleotide - 1231 to -1 as depicted in SEQ ID No. 1 using recombinant DNA methods, thereby augmenting initiation of transcription.

16. A method for preparing riboflavin,
wherein a genetically modified fungus according to one of claims 1 to 5 or 12 to 14 is grown without adding glycine to the culture medium and the riboflavin which is formed is recovered.

17. The method according to claim 16,
wherein the genetically modified fungus according to one of claims 1 to 5 or 12 to 14 is grown with glycine being added to the culture medium.

18. The use of the genetically modified fungus according to one of claims 1 to 5 or 12 to 14 for preparing riboflavin.

19. The use of the nucleotide sequence as depicted in SEQ ID No. 1 encoding a threonine aldolase gene according to one of claims 7 to 9, or of the gene structure according to claim 10, for preparing a genetically modified fungus according to one of claims 1 to 5 or 12 to 14.

20. The use of the vector according to claim 11 for preparing a genetically modified fungus according to one of claims 1 to 5 or 12 to 14.

## Revendications

1. Champignon génétiquement modifié du type Ashbya pour la production de riboflavine par biotechnologie, **caractérisé en ce qu'**il présente suite à sa transformation la séquence de nucléotides selon SEQ ID N° 1 qui code une thréonine aldolase qui présente une forme apte à être répliquée et dont le nombre de copies est supérieur à 1 et **en ce qu'**il présente par expression de ladite séquence de nucléotides une activité catalytique de cette enzyme qui est plus forte que l'activité de la thréonine aldolase endogène.

2. Champignon génétiquement modifié selon la revendication 1, **caractérisé en ce que** sa capacité à synthétiser la riboflavine sans apport externe de glycine est au moins égale à celle d'un type sauvage de l'espèce Ashbya ATCC10895 qui est élevée en conditions normalisées en y ajoutant 6 g/l de glycine externe.

3. Champignon génétiquement modifié selon la revendication 1 ou la revendication 2, **caractérisé en ce que**, dans ce champignon, la décomposition intracellulaire de la glycine est inhibée par l'inhibition de l'activité de la sérinhydroxyméthyle-transférase par la sélection de mutants qui résistent aux antimétabolites de la glycine.

4. Champignon génétiquement modifié selon la revendication 3, **caractérisé en ce qu'**il résiste à l'acide alpha-aminométhylphosphonique ou à l'acide alpha-aminosulfonique, à la β-hydroxynorvaline et/ou à d'autres composants analogues à la thréonine et/ou à la lysine.

5. Champignon génétiquement modifié selon l'une quelconque des revendications 1 à 4, **caractérisé en ce qu'**il s'agit d'un Ashbya gossypii.

6. Thréonine aldolase dont la séquence d'acides aminés est donnée par SEQ ID n° 2.

7. Séquence de nucléotides selon SEQ ID n° 1 qui code une thréonine aldolase.

8. Séquence de nucléotides selon la revendication 7, qui présente une région qui code un gène de thréonine aldolase et qui s'étend des nucléotides 1232 à 2377 selon SEQ ID n° 1.

9. Séquence de nucléotides selon la revendication 7 ou 8, qui présente un promoteur des nucléotides 1 à 1231 placé en amont du gène de l'aldolase de SEQ ID n° 1.

10. Structure de gène qui comprend un gène de la thréonine aldolase qui présente une séquence de nucléotides selon l'une quelconque des revendications 7 à 9 et les séquences de gènes de régulation qui lui sont associées.

11. Vecteur qui comprend un gène de la thréonine aldolase qui présente une séquence de nucléotides selon l'une quelconque des revendications 7 à 9 ou une structure de gène selon la revendication 10.

12. Champignon transformé du type Ashbya, destiné à la production de riboflavine et qui comprend une séquence de nucléotides qui présente une forme apte à être répliquée selon l'une quelconque des revendications 7 à 9 ou une structure de gène selon la revendication 10.

13. Champignon transformé selon la revendication 12, qui. comprend un vecteur selon la revendication 11.

14. Champignon transformé selon la revendication 12 ou la revendication 13, qui est un champignon de l'espèce Ashbya gossypii.

15. Procédé pour produire un champignon génétiquement modifié qui produit de la riboflavine selon la revendication 1 au moyen d'un mode opératoire qui consiste à :
a). augmenter le nombre de copies de gènes chromosomiques ou codés par plasmide de la séquence d'acides nucléiques selon SEQ ID n° 1, de préférence dans le domaine des nucléotides 1 à 1149 selon SEQ ID n° 1 et/ou à
b) remplacer le promoteur par les nucléotides - 1231 à -1 selon SEQ ID n° 1 à l'aide de procédés du génie génétique, ce qui permet d'obtenir un amorçage renforcé de la transcription.

16. Procédé pour produire de la riboflavine, **caractérisé en ce qu'**un champignon génétiquement modifié selon l'une quelconque des revendications 1 à 5 ou 12 à 14 est élevé sans ajout de glycine dans le milieu de culture et **en ce que** la riboflavine formée est traitée.

17. Procédé selon la revendication 16, **caractérisé en ce que** le champignon génétiquement modifié selon l'une quelconque des revendications 1 à 5 ou 12 à 14 est élevé en ajoutant de la glycine dans le milieu de culture.

18. Utilisation du champignon génétiquement modifié selon l'une quelconque des revendications 1 à 5 ou 12 à 14 pour produire de la riboflavine.

19. Utilisation de la séquence de nucléotides selon SEQ ID n° 1 qui code un gène de thréonine aldolase selon l'une quelconque des revendications 7 à 9 ou de la structure de gène selon la revendication 10 pour produire un champignon génétiquement modifié selon l'une quelconque des revendications 1 à 5 ou 12 à 14.

20. Utilisation du vecteur selon la revendication 11 pour produire un champignon génétiquement modifié selon l'une quelconque des revendications 1 à 5 ou 12 à 14.
